Europäisches Patentamt
European Patent Office
Office européen des brevets

(11) Publication number: **0 222 578**
**A2**

# (12) EUROPEAN PATENT APPLICATION

(21) Application number: 86308563.5

(22) Date of filing: 03.11.86

(51) Int.Cl.⁴: A 61 K 37/02

(30) Priority: 06.11.85 US 795566
25.03.86 US 843945

(43) Date of publication of application:
20.05.87 Bulletin 87/21

(84) Designated Contracting States:
CH DE FR GB IT LI NL

(71) Applicant: MERCK & CO. INC.
126, East Lincoln Avenue P.O. Box 2000
Rahway New Jersey 07065(US)

(71) Applicant: MERCK FROSST CANADA INC.
16711 Trans-Canada Highway
Kirkland Quebec(CA)

(72) Inventor: Davies, Philip
24 Essex Road
Scotch Plains New Jersey 07076(US)

(72) Inventor: Moore, Vernon L.
320 East Price Street
Linden New Jersey 07036(US)

(72) Inventor: Ford-Hutchinson, Anthony W.
69 Hyde Park
Beaconsfield Quebec(CA)

(72) Inventor: Slater, Eve E.
19 Kenilworth Drive
Short Hills New Jersey 07078(US)

(74) Representative: Crampton, Keith John Allen et al,
D YOUNG & CO 10 Staple Inn
London WC1V 7RD(GB)

## (54) Use of cyclic somatostatin analogues in the treatment of immediate hypersensitivity diseases.

(57) Cyclic and bridged cyclic somatostatin analogues have been found to be useful in the treatment of diseases manifested by an immediate hypersensitivity response for example, allergic rhinitis, asthma, atopic dermatitis, immediate contact dermatitis and urticaria. The compounds have the formula:

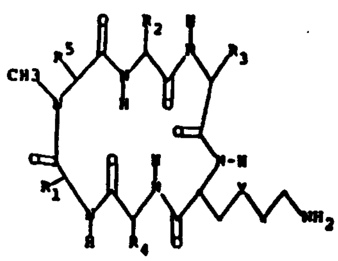

I

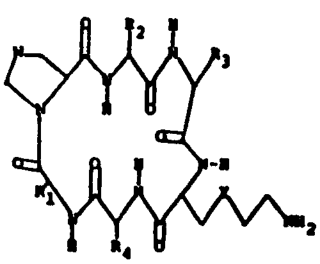

II

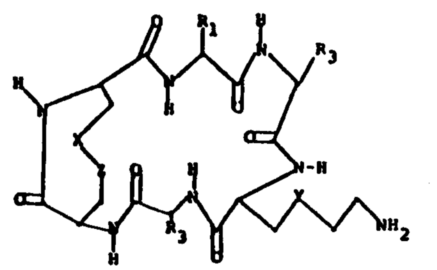

III

H−(D)Phe−Cys−Phe−(D)Trp−Lys−Thr−Cys−Thr−ol
(IV)

where, in the Formulae I, II and III:

W is S or $(CH_2)$ where n is 0, 1, or 2:
one of X and Z is S and the other is S or $CH_2$;
Y is S or $(CH_2)_m$ where m is 0, 1 or 2;
each of $R_1$ and $R_2$, independently of the other, is $C_{1-5}$ alkyl, benzyl, benzyl having one or two $C_{1-5}$ alkyl, halogen, hydroxy, amino, nitro, and/or $C_{1-5}$ alkoxy substituents; or $C_{1-5}$ alkyl substituted with a 5- or 6- membered heterocyclic ring;
$R_3$ is 3-indolylmethyl, either unsubstituted or having $C_{1-5}$ alkyl, $C_{1-5}$ alkoxy or halogen substitution;
$R_4$ is $C_{1-5}$ alkyl, $C_{1-5}$ hydroxyalkyl, benzyl, carboxy($C_{1-5}$

EP 0 222 578 A2

alkyl), amino ($C_{1-5}$ alkyl) or benzyl having a $C_{1-5}$ alkyl, halogen, hydroxy, amino, nitro and/or $C_{1-5}$ alkoxy substituent;

$R_5$ is $C_{1-5}$ alkyl, benzyl, or benzyl having a $C_{1-5}$ alkyl, halogen, hydroxy, amino, nitro, and/or $C_{1-5}$ alkoxy substituent.

# THE TREATMENT OF IMMEDIATE HYPERSENSITIVITY DISEASES

Diseases manifested by an immediate hypersensitivity response include, inter alia, allergic rhinitis, asthma, atopic dermatitis, immediate contact dermatitis and urticaria.

Cyclic and bridge cyclic somatostatin analogues are known compounds. Such compounds and their preparation are described in U.S. Patent Specifications US-A-4,310,518 and 4,235,886, in European Patent Specification EP-A-0 113 209 and in Belgian Patent Specification BE-A-900 089. These documents state that such compounds are capable of inhibiting the release of glucagon, insulin and growth hormone, and of reducing gastric secretions.

The present invention is based on the discovery that diseases manifested by an immediate hypersensitivity response can be treated by the application of such compounds as are disclosed in the above mentioned patent specifications.

Thus, the cyclic and bridged cyclic somatostatin analogue compounds that can be used to treat diseases manifested by an immediate hypersensitivity response and that can be used, in accordance with this invention, in preparing medicaments for those purposes, have the general formula I, II, III or IV:

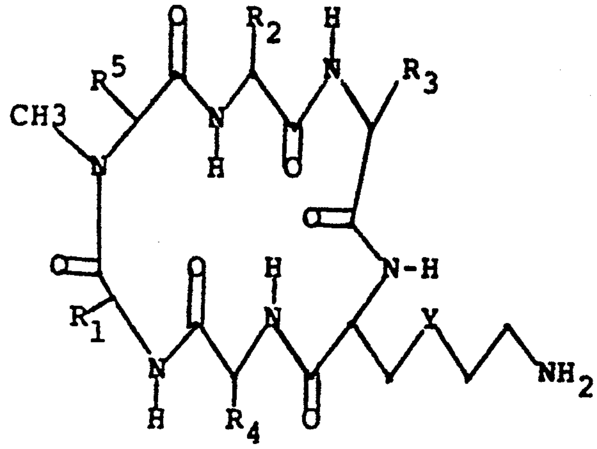

I

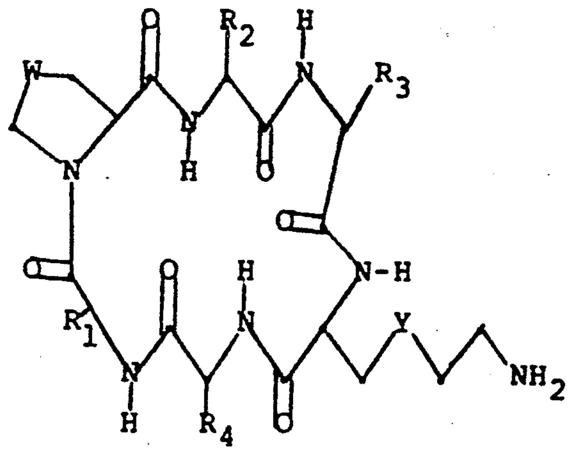

II

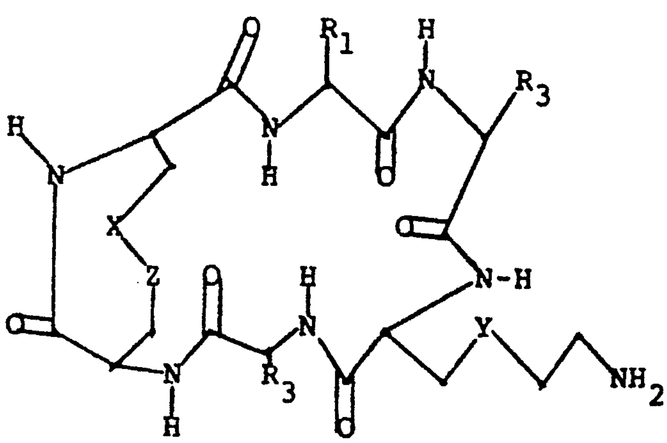

III

H-(D)Phe-Cys-Phe-(D)Trp-Lys-Thr-Cys-Thr-ol

IV

where, in the Formulae I, II and III:

| | |
|---|---|
| W is | S or $(CH_2)_n$ where n is 0, 1, or 2: |
| one of X and Z | is S and the other is S or $CH_2$; |
| Y is | S or $(CH_2)_m$ where m is 0, 1 or 2; |
| each of $R_1$ and $R_2$, | independently of the other, is $C_{1-5}$ alkyl, benzyl, benzyl having one or two $C_{1-5}$ alkyl, halogen, hydroxy, amino, nitro, and/or $C_{1-5}$ alkoxy substituents; or $C_{1-5}$ alkyl substituted with a 5- or 6- membered heterocyclic ring; |
| $R_3$ is | 3-indolylmethyl, either unsubstituted or having $C_{1-5}$ alkyl, $C_{1-5}$ alkoxy or halogen substitution; |
| $R_4$ is | $C_{1-5}$ alkyl, $C_{1-5}$ hydroxyalkyl, benzyl, carboxy-($C_{1-5}$ alkyl), amino ($C_{1-5}$ alkyl) or benzyl having a $C_{1-5}$ alkyl, halogen, hydroxy, amino, nitro and/or $C_{1-5}$ alkoxy substituent; |
| $R_5$ is | $C_{1-5}$ alkyl, benzyl, or benzyl having a $C_{1-5}$ alkyl, halogen, hydroxy, amino, nitro, and/or $C_{1-5}$ alkoxy substituent. |

Examples of $C_{1-5}$ alkyl groups are methyl, ethyl, propyl, iso-propyl, butyl, sec-butyl and pentyl; examples of $C_{1-5}$ alkoxy groups are methoxy, ethoxy, propoxy, isopropoxy, butoxy, tert-butoxy, and pentoxy; halogens are fluorine, chlorine, bromine, or iodine; and the term "5- or 6-membered heterocyclic ring" represents such rings with one or two oxygen, nitrogen and/or sulphur heteroatoms, e.g. imidazole, furan, thiazole, pyrazole and pyridine.

In the compounds of Formulae I, II and III, there are several asymmetric centres which lead to the existence of optical isomers for such compounds. For each of the asymmetric centres of the various amino acids which make up these cyclic hexapeptides, both the D and L configurations are included.

The following are representative cyclic hexapeptide analogues of somatostatin of Formulae I, II and III:

N-Me-Ala-Phe-Trp
Phe-Thr-Lys

Ia

Pro-Phe-Trp
Phe-Thr-Lys

IIa

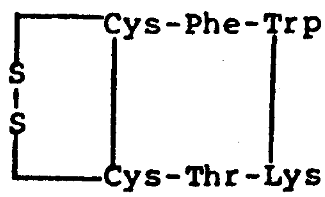

IIIa

Preferred Formula I compounds are:

1) Cyclo-(N-Me-Ala-Tyr-D-Trp-Lys-Thr-Phe)
2) Cyclo-(N-Me-Ala-Phe-D-Trp-Lys-Thr-Phe)
3) Cyclo-(N-Me-Ala-Phe-L-Trp-Lys-Thr-Phe)
4) Cyclo-(N-Me-Ala-Phe-D-Trp-Lys-Thr-p-Cl-Phe)
5) Cyclo-(N-Me-Ala-Phe-D-5-F-Trp-Lys-Thr-Phe)
6) Cyclo-(N-Me-Ala-Phe-L-5-F-Trp-Lys-Thr-Phe)
7) Cyclo-(N-Me-Ala-Phe-D-Trp-Lys-Ser-Phe)
8) Cyclo-(N-Me-Ala-Tyr-D-Trp-Lys-Val-Phe)
9) Cyclo-(N-Me-Ala-Tyr-D-Trp-Lys-Val-Trp)
10) Cyclo-(N-Me-Ala-Tyr-L-Trp-Lys-Val-Phe)
11) Cyclo-(Ser-Ala-N-Me-Phe-His-D-Trp-Lys)

Preferred Formula II compounds are:

12) Cyclo-(Pro-Tyr-D-Trp-Lys-Thr-Phe)
13) Cyclo-(Pro-Phe-D-Trp-Lys-Thr-Phe)
14) Cyclo-(Pro-Phe-L-Trp-Lys-Thr-Phe)
15) Cyclo-(Pro-Phe-D-Trp-Lys-Thr-p-Cl-Phe)
16) Cyclo-(Pro-Phe-D-5-F-Trp-Lys-Thr-Phe)
17) Cyclo-(Pro-Phe-L-5-F-Trp-Lys-Thr-Phe)
18) Cyclo-(Pro-Phe-D-Trp-Lys-Ser-Phe)

Preferred Formula III compounds are:

19) Cyclo-(Cys-Cys-Tyr-D-Trp-Lys-Thr)
20) Cyclo-(Cys-Cys-Tyr-D-Trp-Lys-Val)
21) Cyclo-(Cys-Cys-Tyr-L-Trp-Lys-Val)
22) Cyclo-(Cys-Cys-Phe-D-Trp-Lys-Thr)
23) Cyclo-(Cys-Cys-Phe-L-Trp-Lys-Thr)
24) Cyclo-(Cys-Cys-His-D-Trp-Lys-Thr)
25) Cyclo-(Cys-Cys-His-D-Trp-Lys-Val)
26) Cyclo-(Cys-Cys-Aha-Phe-D-Trp-Lys-Thr)

The abbreviated designations for the amino acid components are given below:

| Abbreviated Designation | Amino Acid |
|---|---|
| Lys | L-lysine |
| Phe | L-phenylalanine |
| Trp | L-tryptophan |
| D-Trp | D-tryptophan |
| Thr | L-threonine |
| Aha | 7-aminoheptanoic acid |
| Tyr | L-tyrosine |
| Val | L-valine |
| Abu | L-α-aminobutyric acid |
| Ser | L-serine |
| Asn | L-asparagine |
| Pro | L-proline |
| Asu | D- or L-aminosuberic acid |
| Cys | L-cysteine |

Treatment of diseases manifested by an immediate hypersensitivity response with the compounds of Formula I, II, III or IV is accomplished by providing a suitable pharmaceutical composition containing one or more such compounds as the active ingredient.

Thus, suitable pharmaceutical compositions containing the active ingredient can be in such forms as creams, ointments, jellies, solutions, suspensions, nasal or inhalant sprays or drops, eye drops, and pressurized or non-pressurized dispersible powders, and they can be used to effectively treat warm-blooded animals such as mice, rats, horses, dogs, cats, cattle and humans. Such pharmaceutical compositions, in addition to an effective dosage amount of the active ingredient, typically include pharmaceutically acceptable carriers, adjuvants and vehicles.

For example, aqueous suspensions can be used containing the active ingredient in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents, for example, a naturally occuring phosphatide, for example lecithin, or a condensation product of an alkylene oxide with a fatty acid, for example polyoxyethylene stearate, or a condensation product of ethylene oxide with long chain aliphatic alcohols, for example, heptadecaethyleneoxycetanol, or a condensation product of ethylene oxide with partial esters derived from fatty acids and a hexitol such as polyoxyethylene sorbitol monooleate, or a condensation product of ethylene oxide with partial esters derived from fatty acids and hexitol anhydrides, for example polyoxy-ethylene sorbitan monooleate. The aqueous suspensions may also contain one or more preservatives, for example, ethyl or n-propyl p-hydroxybenzoate, one or more colouring agents, one or more flavouring agents, and one or more sweetening agents, such as sucrose or saccharin.

Oily suspensions can also be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents, such as those set forth above, and flavouring

agents may be added to provide a palatable oral preparation. These compositions may be preserved by the addition of an antioxidant such as ascorbic acid.

Pressurized or non-pressurized dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavouring and colouring agents, may also be included.

The pharmaceutical composition of the present invention may also be in the form of an oil-in-water emulsion. The oily phase may be a vegetable oil, for example olive oil or arachis oil, or a mineral oil, for example liquid paraffin, or mixtures of these. Suitable emulsifying agents may be naturally-occurring gums, for example gum acacia or gum tragacanth, naturally-occurring phosphatides, for example soy bean and lecithin, and esters or partial esters derived from fatty acids and hexitol anhydrides, for example sorbitan monooleate, and condensation products of the said partial esters with ethylene oxide, for example polyoxyethlene sorbitan monooleate. The emulsions may also contain sweetening and flavouring agents.

The pharmaceutical compositions can be in the form of an oleaginous suspension, which can be formulated according to the known art using suitable dispersing or wetting agents and suspending agents that have been mentioned above.

For topical use, formulations such as creams, ointments, jellies, solutions and suspensions containing the anti-inflammatory agents are used.

The amount of active ingredient, i.e. the compound of Formula I, II, III, or IV or a mixture of two or more such compounds, will vary depending, for example, the condition being treated and the size and kind of mammal. Generally speaking, the active ingredient can be used in any amount capable of treating immediate hypersensitivity diseases as well as at doses one-fifth to one-third lower than the usual amounts for multiple daily applications.

For humans, typical effective amounts of active ingredient for use in unit dose compositions of the invention are 0.001% to 2.0% by weight of the composition, preferably 0.1% to 0.5% by weight of the composition. However, greater amounts can be used if desired or prescribed.

EXAMPLE

To demonstrate the use of the compounds of the invention to treat diseases manifested by an immediate hypersensitivity response, a compound was selected as representative of the compounds of the invention and tested in rats. The compound used was compound 8) of the Formula I compounds having the formula:

8) Cyclo-(N-Me-Ala-Tyr-D-Trp-Lys-Val-Phe) which is identified in the following description as "Form.I-8)".

INTRODUCTION

Sensitized inbred "asthmatic rats" challenged with aerosolized antigen respond with symptoms of continuous dyspnea which is partly mediated by serotonin. The serotonin component can be completely blocked by prior treatment with 3 μg/kg of methysergide administered i.v. before challenge. These rats differed from normal rats in that they have nonspecific bronchial hyperreactivity to a number of bronchoconstrictor agents [Brunet *et al.*, *J. Immun.*, 131, 434-438 (1983)].

It is known that pretreatment with drugs used in the clinical management of asthma decrease the duration of continuous dyspnea and, at higher

doses, eliminate the response [Piechuta et al., Immun., 38, 385-392 (1979)].

METHODS

(1) Animals: Rats were obtained from an inbred line of asthmatic rats that were developed and maintained in local laboratories and later at a local farm. Male and female rats were used between 10-12 weeks of age. Their weights ranged from 220-240 gm (females) and 350-400 gm (males). Rats were matched by sex and littermates between the various treatment groups.

(2) Materials: Egg albumin (EA), grade V, crystallized and lyophilized (Sigma Chemical Co., St. Louis), aluminum hydroxide (Reheis Chemical Company, Chicago), and methysergide bimaleate (Sandoz Ltd., Basel, Switzerland).

(3) Apparatus: The challenge and subsequent respiratory recordings were carried out in a clear plastic box with internal dimensions 10 x 6 x 4 inches. The top of the box was removable; in use, it was held firmly in place by four clamps and an airtight seal was maintained by a soft rubber gasket. Through the centre of each end of the chamber a Devilbiss nebulizer (No. 40) was inserted via an airtight seal and each end of the box was also equipped with an outlet. A Fleisch No. 0000 pneumotachograph was inserted into one end of the box and coupled to a Grass volumetric pressure transducer (PT5-A) which was then connected to a Beckman Type R Dynograph through appropriate couplers. While

aerosolizing the antigen, the outlets were open and the pneumotachograph was isolated from the chamber. The outlets were closed and the pneumotachograph and the chamber were connected during the recordings of the respiratory patterns. For challenge, 2 ml of 3% solution of antigen in saline was placed into each nebulizer and the aerosol was generated with air from a small Potter diaphragm pump operating at 10 psi and a flow of 8 litre/minute.

(4) Procedure: Rats were sensitized by injecting (s.c.) 1 ml of suspension containing 1 mg egg albumin (EA) and 200 mg aluminum hydroxide in saline. They were used on day 13 post sensitization. Rats were exposed to aerosol of 3% EA in saline for exatly 1 minute, then their respiratory profiles were recorded for an additional 30 minutes. The duration of continuous dyspnea was measured from the respiratory recordings.

Form.I-8) was administered subcutaneously as a solution in 5% mannitol 1 hour prior to challenge with antigen. Methysergide bimaleate was dissolved in saline and given intravenously at dose of 3 μg/kg 5 minutes before challenge. Dose volume employed for subcutaneous treatments was 2 ml/kg and for i.v. treatments 1 ml/kg. Control groups of rats (n=6) were treated subcutaneously with drug vehicle (5% mannitol).

The sensitivity of various drug treatments were determined in terms of the decrease in duration of dyspnea in comparison with a group of vehicle-treated controls. Comparisons between groups were made using the Student's t-test.

RESULTS

Groups of 6 sensitized inbred rats were treated subcutaneously with 1 mg/kg of Form.I-8) alone or in combination with methysergide. The results shown in Table 1 below reveal that Form.I-8) evaluated in the presence of methysergide produced significant inhibition (57% $p < 0.01$) of the duration of antigen-induced dyspnea. However, without methysergide to block the actions of the serotonin component of the response, Form.I-8) failed to produce activity in these rats.

**TABLE 1**

Effect of Form.I-8) on Antigen-Induced Dyspnea in Inbred Rats

| Treatment | Duration of Dyspnea in Minutes (Mean±S.D.) n=6/group | Inhibition % |
|---|---|---|
| Control Group - Vehicle s.c. 1 hour (+ Methysergide)* | 13.4 ± 4.3 | |
| Form.I-8) - 1 mg/kg s.c. 1 hour (+ Methysergide)* | 5.7 ± 3.9 | 57 $p < 0.01$ |
| Control Group - Vehicle s.c. 1 hour (- Methysergide) | 18.1 ± 2.8 | |
| Form.I-8) - 1 mg/kg s.c. 1 hour (- Methysergide) | 18.2 ± 3.6 | 0 |

* Methysergide (3 μg/kg) was administered intravenously 5 minutes prior to challenge with antigen.

## CLAIMS

1. For use in the manufacture of a medicament for treating diseases manifested by an immediate hypersensitivity response, a compound having the Formulae:

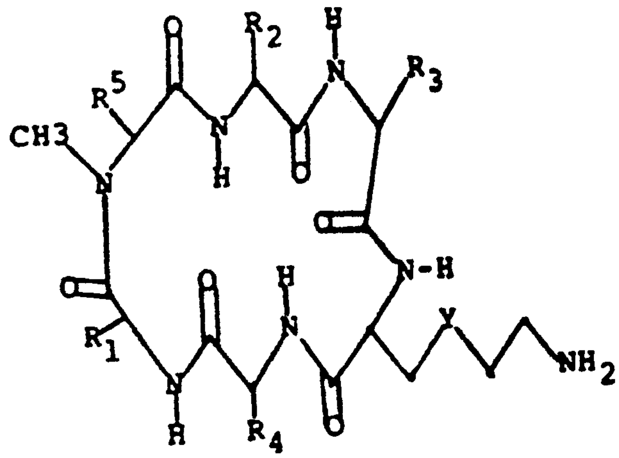

I

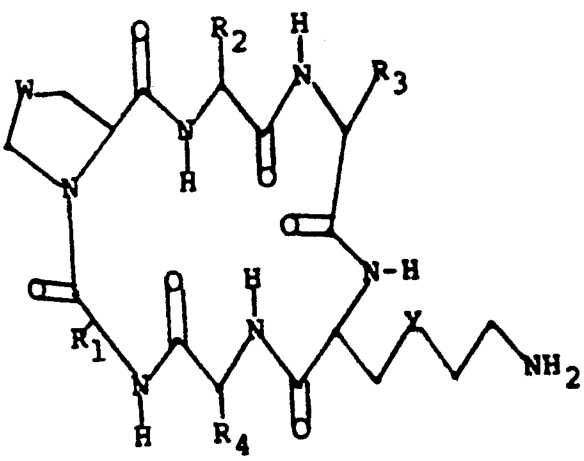

II

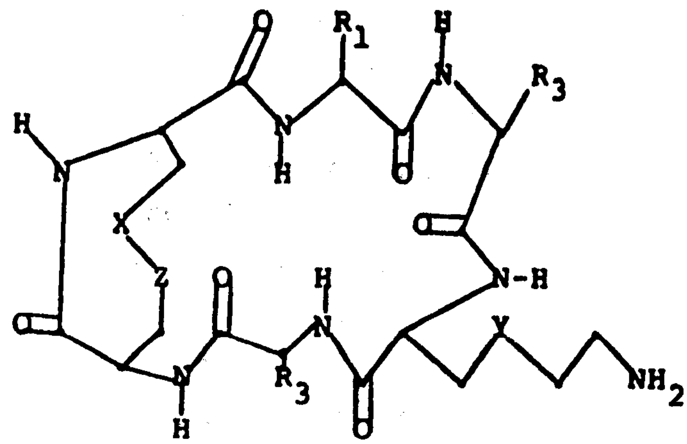

III

H-(D)Phe-Cys-Phe-(D)Trp-Lys-Thr-Cys-Thr-ol

IV

where, in the Formulae I, II and III:

| | |
|---|---|
| W is | S or $(CH_2)_n$ where n is 0, 1, or 2: |
| one of X and Z | is S and the other is S or $CH_2$; |
| Y is | S or $(CH_2)_m$ where m is 0, 1 or 2: |
| each of $R_1$ and $R_2$, | independently of the other, is $C_{1-5}$ alkyl, benzyl, benzyl having one or two $C_{1-5}$ alkyl, halogen, hydroxy, amino, nitro, and/or $C_{1-5}$ alkoxy substituents; or $C_{1-5}$ alkyl substituted with a 5- or 6- membered heterocyclic ring; |
| $R_3$ is | 3-indolylmethyl, either unsubstituted or having $C_{1-5}$ alkyl, $C_{1-5}$ alkoxy or halogen substitution: |
| $R_4$ is | $C_{1-5}$ alkyl, $C_{1-5}$ hydroxyalkyl, benzyl, carboxy-($C_{1-5}$ alkyl), amino ($C_{1-5}$ alkyl) or benzyl having a $C_{1-5}$ alkyl, halogen, hydroxy, amino, nitro and/or $C_{1-5}$ alkoxy substituent; |
| $R_5$ is | $C_{1-5}$ alkyl, benzyl, or benzyl having a $C_{1-5}$ alkyl, halogen, hydroxy, amino, nitro, and/or $C_{1-5}$ alkoxy substituent. |

2. Cyclo-(N-Me-Ala-Tyr-D-Trp-Lys-Thr-Phe);
Cyclo-(N-Me-Ala-Phe-D-Trp-Lys-Thr-Phe);
Cyclo-(N-Me-Ala-Phe-L-Trp-Lys-Thr-Phe);
Cyclo-(N-Me-Ala-Phe-D-Trp-Lys-Thr-p-Cl-Phe);
Cyclo-(N-Me-Ala-Phe-D-5-F-Trp-Lys-Thr-Phe);
Cyclo-(N-Me-Ala-Phe-L-5-F-Trp-Lys-Thr-Phe);
Cyclo-(N-Me-Ala-Phe-D-Trp-Lys-Ser-Phe);
Cyclo-(N-Me-Ala-Tyr-D-Trp-Lys-Val-Phe);
Cyclo-(N-Me-Ala-Tyr-D-Trp-Lys-Val-Trp);
Cyclo-(N-Me-Ala-Tyr-L-Trp-Lys-Val-Phe); and
Cyclo-(Ser-Ala-N-Me-Phe-His-D-Trp-Lys),
for use in the manufacture of a medicament for treating diseases manifested by an immediate hypersensitivity response.

3. Cyclo-(Pro-Tyr-D-Trp-Lys-Thr-Phe);
Cyclo-(Pro-Phe-D-Trp-Lys-Thr-Phe);
Cyclo-(Pro-Phe-L-Trp-Lys-Thr-Phe);
Cyclo-(Pro-Phe-D-Trp-Lys-Thr-p-Cl-Phe);
Cyclo-(Pro-Phe-D-5-F-Trp-Lys-Thr-Phe);
Cyclo-(Pro-Phe-L-5-F-Trp-Lys-Thr-Phe); and
Cyclo-(Pro-Phe-D-Trp-Lys-Ser-Phe),
for use in the manufacture of a medicament for treating diseases manifested by an immediate hypersensitivity response.

4. Cyclo-(Cys-Cys-Tyr-D-Trp-Lys-Thr);
Cyclo-(Cys-Cys-Tyr-D-Trp-Lys-Val);
Cyclo-(Cys-Cys-Tyr-L-Trp-Lys-Val);
Cyclo-(Cys-Cys-Phe-D-Trp-Lys-Thr);
Cyclo-(Cys-Cys-Phe-L-Trp-Lys-Thr);
Cyclo-(Cys-Cys-His-D-Trp-Lys-Thr);
Cyclo-(Cys-Cys-His-D-Trp-Lys-Val); and
Cyclo-(Cys-Cys-Aha-Phe-D-Trp-Lys-Thr),
for use in the manufacture of a medicament for treating diseases manifested by an immediate hypersensitivity response.

5. For use in the treatment of diseases by an immediate hypersensitivity response, a compound of Formula I, II, III or IV as claimed in Claim 1.